# EUROPEAN PATENT SPECIFICATION

(11) **EP 1 222 027 B1**
(45) Date of publication and mention of the grant of the patent: **13.07.2005**
(21) Application number: 00967347.6
(22) Date of filing: 06.10.2000
(51) Int. Cl.: B01J 37/03, B01J 37/08, B01J 29/06, C07C 1/04

(54) **PREPARATION OF CATALYSTS FOR FISCHER-TROPSCH HYDROCARBON SYNTHESIS**
HERSTELLUNG VON KATALYSATOREN FÜR DIE FISCHER-TROPSCH KOHLENWASSERSTOFFSYNTHESE
PREPARATION DE CATALYSEURS POUR LA SYNTHESE FISCHER-TROPSCH D'HYDROCARBURES

(30) Priority: 12.10.1999 US 416028
(43) Date of publication of application: 17.07.2002
(73) Proprietor: ExxonMobil Research and Engineering Company, Annandale, New Jersey 08801 (US)
(72) Inventor: LAPIDUS, Albert L'vovich, Moscow, 115522 (RU); KRYLOVA, Alla Jurievna, Moscow, 109518 (RU); DAAGE, Michel, A., Baton Rouge, LA 70810 (US); KOVEAL, Russell, John, Baton Rouge, LA 70817 (US)
(74) Representative: Troch, Geneviève
(86) International application number: PCT/US2000/027715
(87) International publication number: WO 2001/026810

(56) References cited:
- US-A- 2 850 515
- US-A- 4 086 262
- US-A- 4 659 743
- US-A- 4 849 571

## Description

### FIELD OF THE INVENTION

The invention relates to the preparation of catalysts containing a zeolite component, which are useful for Fischer-Tropsch hydrocarbon synthesis. More particularly, the invention relates to preparing a hydrocarbon synthesis catalyst by a process which comprises precipitating a catalytic metal component in aqueous media with a carbonate, with the resulting slurry heated to drive out CO₂ and form a CO₂-reduced slurry comprising the precipitate, followed by compositing the precipitate with a zeolite component to form a catalyst precursor, which is then dried and reduced, to form the catalyst.

### BACKGROUND OF THE DISCLOSURE

The synthesis of hydrocarbons from a synthesis gas comprising a mixture of H₂ and CO is well known. The synthesis gas feed is contacted with a particulate Fischer-Tropsch type of catalyst at conditions effective for the H₂ and CO in the feed gas to react and form hydrocarbons. The reaction is conducted in a fixed or fluidized catalyst bed reactor or in a slurry reactor. The catalysts are also well known and typically include a composite of at least one iron group catalytic metal component composited with at least one inorganic refractory metal oxide material, such as alumina, amorphous, silica-alumina and the like. Various catalyst preparation methods have been used to form hydrocarbon synthesis catalysts. For example, US patent 2,850,515 discloses a Fischer-Tropsch catalyst prepared by immersing a dehydrated, acid-refined clay into a metal precipitate, formed by adding an aqueous solution comprising cobalt and magnesium nitrates to an aqueous solution of sodium carbonate. The resulting impregnate is dried and reduced in hydrogen prior to use. US 3,013,990 discloses a hydrogenation catalyst preparation in which a zeolite is ion exchanged with iron, cobalt or nickel, then reduced in hydrogen, followed by heating to dehydrate the composite to form an activated catalyst. US 4,086,262 discloses a hydrocarbon synthesis having a zeolite component, wherein the zeolite, in either an ammonium or ion-exchanged form, is either composited or impregnated with one or more catalytic metal components, and then calcined prior to use. The present conventional method of catalyst preparation for a Fischer-Tropsch catalyst is to composite the catalytic metal component with a zeolite by ion exchange, impregnation or from a molten salt, after which the resulting composite is reduced and calcined or reduce, calcined and reduced again, to form the active catalyst. For example, US patent 4,492,774 discloses preparing a Fischer-Tropsch catalyst by impregnating a zeolite with a catalytic metal salt, followed by drying and calcining. The impregnation-drying and calcining cycle is repeated a multiple number of times, until the desired amount of catalytic metal loading is achieved. The final calcined composite is then activated by a cycle of reduction-oxidation-reduction, to form the activated catalyst.

US 4,659,743 discloses a Fischer-Tropsch catalyst consisting essentially of a mixture of cobalt, thoria and a crystalline zeolite obtained by precipitation of the metals as carbonate from a solution of cobalt and thorium salts, drying of the precipitate and calcinate to their oxides, blending the said precipitate in a physical mixture with the zeolite in granular form. Alternatively, a solution of the cobalt and thorium soluble salts is used to permeate into the zeolite which is subsequently dried and heated to a sufficient temperature to convert the catalytic metals to the oxide. In either of these processes, the cobalt oxide dispersed throughout the zeolite is reduced before use.

### SUMMARY OF THE INVENTION

The invention relates to the preparation of catalysts useful for synthesizing hydrocarbons from a synthesis gas comprising a mixture of H₂ and CO, such as Fischer-Tropsch hydrocarbon synthesis catalysts. These catalysts are prepared by a process which comprises (i) precipitating, with a carbonate ion precipitant under mixing conditions, at least one catalytic metal component as a hydroxy metal carbonate, from a catalytic metal salt solution in aqueous media, to form a first slurry (ii) heating the first slurry to remove CO₂ and form a CO₂-reduced second slurry, in which the hydroxy metal carbonate precipitate is different from that formed in step (i) above, (iii) compositing the precipitate from the second slurry with a zeolite component to form a catalyst precursor, which is then (iv) reduced to form the catalyst. Driving off the CO₂ alters the structure of the precipitated catalytic metal component, so that its structure is different after CO₂ removal, from what it was before CO₂ removal. The precursor is dried prior to reduction and preferably dried to a moisture content of no less than 6 wt. % moisture. More preferably the precursor will have a moisture content of from 6-15 wt. % and most preferably from 8-12 wt. %, prior to reduction. The dry precursor is reduced with hydrogen or a hydrogen-containing reducing gas to form the catalyst. The one or more zeolite components of the catalyst are preferably dehydrated prior to being composited with the catalytic metal precipitate resulting after the CO₂ removal. When used in a Fischer-Tropsch type of hydrocarbon synthesis process, these catalysts have been found to have excellent stability and activity maintenance, with high CO conversion and C₅₊ hydrocarbon yield.

The structure of a hydroxy metal carbonate precipitate resulting from using a carbonate ion precipitant, and the catalytic properties of a catalyst derived therefrom, are highly influenced by the exact procedure used to form the catalytic metal precipitate. In the process of forming the catalyst according to the practice of the invention, a carbonate ion precipitant is added to an aqueous solution of at least one catalytic metal salt, to precipitate the catalytic metal as a first hydroxy metal carbonate and form a first slurry, followed by heating the slurry to remove CO₂ and form a CO₂-reduced, second slurry containing a second hydroxy metal carbonate. The second hydroxy metal carbonate is different from that which existed prior to the CO₂ removal. The zeolite component(s) of the catalyst is composited with the precipitate after the CO₂ removal, to form the catalyst precursor, which is then dried and reduced as above, to form the catalyst. The catalyst is then loaded into a hydrocarbon synthesis reactor for synthesizing hydrocarbons. In the embodiment in which the catalyst is used for hydrocarbon synthesis, the process comprises contacting a synthesis gas comprising a mixture of H₂ and CO with a hydrocarbon synthesis catalyst at synthesis reaction conditions sufficient for the H₂ and CO to react and form hydrocarbon products, wherein said catalyst comprises at least one catalytic metal component and at least one zeolite component formed by (i) adding a carbonate ion precipitant to an aqueous solution of at least one catalytic metal salt, to precipitate the catalytic metal as a first hydroxy metal carbonate and form a first slurry, followed by (ii) heating the slurry to remove CO₂ and form a CO₂-reduced, second slurry containing a second hydroxy metal carbonate, (iii) compositing the second hydroxy metal precipitate with a zeolite component to form a catalyst precursor, (iv) drying the precursor to a moisture content of at least 6 wt. %, and (v) reducing the precursor with hydrogen to form the catalyst.

Typically, at least a portion of the synthesized hydrocarbons are upgraded by one or more upgrading operations which comprise fractionation and/or conversion in which at least a portion of the molecular structure of the hydrocarbons is changed. By hydrocarbons is meant to include oxygenated, olefinic and paraffinic hydrocarbons, with the paraffinic hydrocarbons preferably comprising C₅₊ and more preferably C₁₀₊ paraffins.

### DETAILED DESCRIPTION

As mentioned above, the exact procedure and conditions employed to form a hydroxylated or hydroxy metal carbonate precipitate, from an aqueous metal salt solution using a carbonate ion as a precipitant, has a strong influence on the structure of the precipitate and properties of the resulting catalyst. This is known and disclosed, for example, in an article by F. Cavani, et al., "Hydrotalcite-Type Anionic Clays: Preparation, Properties and Applications, on pages 173-215 of Catalysis Today, 11 (Elsevier, 1991). While not wishing to be held to any particular theory, it is believed that precipitating out the metal component(s) with a carbonate ion, produces a precipitate comprising a hydroxy metal carbonate having a hydrotalcite or hydrotalcite-like structure, according to Caviani, et al. The structure and carbon content of this hydroxy metal carbonate is believed to be altered both by driving CO₂ out of the resulting slurry and by the elevated temperature employed by the heating used to remove the CO₂ from the slurry, based on the performance characteristics and stability of the resulting catalyst. However, irrespective of any theory, it has been found that the properties of the final catalyst are significantly affected by these variables. The one or more catalytic metal component precursor salts will comprise one or more water-soluble inorganic or organic salts, such as a nitrate, acetate, formate and the like. Sulfur, phosphorus, halides and alkali metal-containing salts are to be avoided. The nitrates are often the salt of choice to the practitioner. The anion portion of the salt or salts must be decomposable at the reducing conditions used to form the catalyst from its precursor and leave no undesirable residues as a result of decomposition or adversely interfere with the reduction. In addition to containing one or metal salts of one or more catalytic metal components of the catalyst, the aqueous catalytic metal salt solution may also contain a metal salt of one or more promoter metals, such as magnesium (e.g., Mg(NO₃)₂*6H₂O), zirconium (e.g., ZrO(NO₃)₂*2H₂O) and the like. In this case the metals are coprecipitated and the resulting precipitate my comprise one or more bi- or multimetal hydroxy carbonates. Alternately, one or more promoter metal oxides may be composited, along with the one or more zeolite components, with the catalytic metal precipitate remaining after CO₂ removal from the slurry. Thus, the aqueous catalytic metal component salt solution may contain salts of one or more catalytic metals, along with one or more salts of one or more catalytic promoters.

In the practice of the invention, the carbonate ion precipitant is added to the aqueous catalytic metal salt solution, under mixing conditions, to form the hydroxy metal carbonate precipitate. By mixing conditions is meant agitation or mixing sufficient to form an intimate mixture of the source of carbonate ion and salt solution, during the precipitation. The aqueous carbonate and/or bicarbonate salt solution may comprise more than one carbonate and/or bicarbonate salts, with the cation being one or more metals, preferably alkali metal, and/or ammonium ion, depending on the desired catalyst. While carbonate or bicarbonate salts may also be used, carbonates are preferred. The aqueous carbonate solution will typically comprise from 1 to 20 wt. % of one or more ammonium or alkali carbonates. It is important to the practice of the invention, that the sequence of adding the aqueous carbonate salt solution to the aqueous catalytic metal component salt solution to form the precipitate be followed. A catalyst of the invention will not be formed, if the catalytic metal salt solution is added to the carbonate solution.

In the process of the invention, after the hydroxy metal carbonate has been precipitated by the carbonate ion to form a first slurry, the resulting aqueous slurry containing the precipitate must be heated at an elevated temperature of preferably greater than (150°F) 65°C, to drive out and remove CO₂. The exact time and temperature may vary, along with the pressure, but it has been found satisfactory to heat the slurry at its boiling point. Heating is preferably maintained until CO₂ evolution ceases. The heating time at a given temperature and pressure must be determined experimentally. As mentioned above, this alters the form of the precipitated hydroxy metal carbonate and results in a CO₂-reduced, second slurry. It is this altered precipitate that is composited with the one or more zeolite components of the catalyst.

The one or more zeolites in particulate form may be composited with the precipitate resulting from the CO₂ removal by any well known methods. Thus, the precipitate may be separated from the slurry liquid by filtration or other separation means, to form a paste which is then intimately mixed and kneaded with the zeolite(s) and one or more other components, such as one or more promoter or support components, to form a catalyst precursor composite. More typically, at least one zeolite component of the catalyst, in particulate form, is composited with the precipitate, by contacting it, in particulate form, with the precipitate in the second slurry, under mixing conditions to form an intimate mixture of precipitate and zeolite, followed by filtration to form a paste. The paste is then extruded to the desired shape and size and dried. The drying is conducted at a temperature (e.g., 105-125°C) and for a time (e.g., 50-250 minutes) sufficient for the resulting catalyst precursor to have a moisture content of at least 6 wt. %. The precursor is then reduced, typically in flowing hydrogen or a hydrogen-containing reducing gas, at an elevated temperature sufficient to achieve reduction of the metal. It is preferred that the precursor contain at least 6 wt. % H₂O and preferably no more than 15 wt. % H₂O, prior to reduction, to form a more active catalyst. This is based on experimental results. In determining the residual moisture content of catalyst precursors made according to the process of the invention, a sample of washed and dried extrudate was placed in a drying oven in air, for varying times at 105-110°C to drive off the residual moisture, until there was no change in weight. This was taken as zero moisture content. Actual times varied from 65 to 170 minutes. The residual moisture content of the precursor extrudate was determined by the difference between the constant weight, of which there was no further change in with increased drying time, and the weight of the precursor containing the residual moisture, prior to the further drying to constant weight. The moisture-containing precursor was then reduced with hydrogen as set forth below. The hydrogen-containing reducing gas may be all hydrogen, but will more typically comprise a mixture of hydrogen and any suitable inert gas. In this context, an inert gas is any gas which doesn't interfere with or affect the reduction, or alter the properties of the catalyst, and may comprise light hydrocarbons, argon, and the like. The reducing conditions include a pressure in the range of from 0.1 to 100 atm., preferably 1 to 50 atm. and more preferably from 1 to 10 atm. The reducing temperature will broadly range from 200 to 600°C, preferably from 300 to 450°C and more preferably from 325 to 450°C. The hydrogen reducing gas will comprise at least 10 vol. % of hydrogen.

In the practice of the invention, the one or more zeolite components of the catalyst will include shape selective molecular sieves which, when combined with at least one catalytic metal component, will be useful for the desired catalyst process for which the catalyst is intended. By zeolite is meant both natural and synthetic aluminosilicate molecular sieves, including but not limited to the A, X, Y, mordenite and ferrierite types, in both single and multivalent cation forms as well as the hydrogen forms and including dealuminized forms. The mole ratio of the silica to the alumina may range from 2 to 400 and the pore size may range from 4 to 15. Such zeolites include, for example, in addition to ferrierite, and mordenite types, ZSM-5, ZSM-11, ZSM-23, ZSM-35, ZSM-48, ZSM-22 also known as theta one or TON, Beta, MCM, L and the silicoaluminophosphates known as SAPO's. The zeolite component is preferably dehydrated prior to contacting the aqueous slurry. Further, the zeolite component may be composited with binder such as alumina, clay and the like, prior to contact with the catalytic metal-containing precipitate. Preferred zeolites include at least one of a Y zeolite, an HY zeolite and a HZSM-5 zeolite. In some cases it is preferred that the zeolite be stabilized by being dealuminized, by any suitable method known to those skilled in the art.

The catalytic metal loading of the final catalyst will range, for cobalt as an example, from 5 to 70 wt. % and preferably from 20-40 wt. % of the total catalyst composition following reduction. Additional catalyst components may be added to or composited with the other catalytic components in conventional manners, either before the precipitation. Thus, and as mentioned above, one or more promoter metal salts of, for example, Zr, Mg, Th, Ti and the like may be coprecipitated with the one or more catalytic metal components. Alternately, one or more promoters and other components may be added to the precursor composite containing the second precipitate, subsequent to the precipitation or before or subsequent to, drying, but prior to reduction to form the catalyst. These may include, for example, one or more oxide promoters such as ZrO₂, ThO₂, TiO₂ and like, typically in powdered form, as well as support components and binders. Such promoters will typically range from 0.1 to 20 wt. % of the catalyst composition, following reduction. While the catalytic metal and promoter metal components of the catalyst will depend on the process, suitable catalytic metals for a Fischer-Tropsch hydrocarbon synthesis process catalyst comprise, for example, one or more Group VIII metals such as Fe, Ni, Co and Ru and, in some cases, will comprise catalytically effective amounts of one or more Group VIII metals, such as Co, and one or more promoter metal components. Suitable promoter metals include Re, Ru, Fe, Ni, Th, Zr, Hf, U, Mg and La, composited with the zeolite component and one or more other suitable inorganic support materials, preferably which comprise one or more refractory metal oxides. The metal Groups referred to herein are those found in the Sargent-Welch Periodic Table of the Elements, © 1968. Titania is a useful component, particularly when employing a slurry hydrocarbon synthesis process in which higher molecular weight, primarily paraffinic liquid hydrocarbon products are desired.

The hydrocarbons synthesized by a hydrocarbon synthesis process employing a catalyst prepared according to the practice of the invention are typically upgraded to more valuable products, by subjecting all or a portion of the hydrocarbon products produced in the synthesis reactor to one or more upgrading operations which include fractionation and/or one or more conversion operations. By conversion is meant one or more operations in which the molecular structure of at least a portion of the hydrocarbon is changed and includes both noncatalytic processing (e.g., steam cracking), and catalytic processing (e.g., catalytic cracking) in which a fraction is contacted with a suitable catalyst. If hydrogen is present as a reactant, such process steps are typically referred to as hydroconversion and include, for example, hydroisomerization, hydrocracking, hydrodewaxing, hydrorefining and the more severe hydrorefining referred to as hydrotreating, all conducted at conditions well known in the literature for hydroconversion of hydrocarbon feeds, including hydrocarbon feeds rich in paraffins. Illustrative, but nonlimiting examples of more valuable products formed by conversion include one or more of a synthetic crude oil, liquid fuel, olefins, solvents, lubricating, industrial or medicinal oil, waxy hydrocarbons, nitrogen and oxygen containing compounds, and the like. Liquid fuel includes one or more of motor gasoline, diesel fuel, jet fuel, and kerosene, while lubricating oil includes, for example, automotive, jet, turbine and metal working oils. Industrial oil includes well drilling fluids, agricultural oils, heat transfer fluids and the like.

With respect to the hydrocarbon synthesis, fixed bed, fluid bed and slurry hydrocarbon synthesis processes for forming hydrocarbons from a synthesis gas comprising a mixture of H₂ and CO are well known and documented in the literature. In all of these processes, the synthesis gas is reacted in the presence of a suitable Fischer-Tropsch type of hydrocarbon synthesis catalyst, at reaction conditions effective to form hydrocarbons. Except for a fluidized bed process in which the synthesis hydrocarbons are all gaseous at the reaction conditions, some of these synthesized hydrocarbons will be liquid, some solid (e.g., wax) and some gas at standard room temperature conditions of temperature and pressure of 25°C and one atmosphere, particularly if a catalyst having a catalytic cobalt component is used. Slurry processes are often preferred because of their superior heat (and mass) transfer characteristics for the strongly exothermic synthesis reaction and because they are able to produce relatively high molecular weight, paraffinic hydrocarbons when using a cobalt catalyst. In a slurry HCS process a synthesis gas comprising a mixture of H₂ and CO is bubbled up as a third phase through a slurry in a reactor which comprises a particulate Fischer-Tropsch type hydrocarbon synthesis catalyst dispersed and suspended in a slurry liquid comprising hydrocarbon products of the synthesis reaction which are liquid at the reaction conditions. The mole ratio of the hydrogen to the carbon monoxide may broadly range from about 0.5 to 4, but is more typically within the range of from about 0.7 to 2.75 and preferably from about 0.7 to 2.5. The stoichiometric mole ratio for a Fischer-Tropsch hydrocarbon synthesis reaction is 2.0, but in the practice of the present invention it may be increased to obtain the amount of hydrogen desired from the synthesis gas for other than the HCS reaction. In a slurry process the mole ratio of the H₂ to CO is typically about 2.1/1. Slurry process conditions vary somewhat depending on the catalyst and desired products. Typical conditions effective to form hydrocarbons comprising mostly C₅₊ paraffins, (e.g., C₅₊-C₂₀₀) and preferably C₁₀₊ paraffins, in a slurry HCS process employing a catalyst comprising a supported cobalt component include, for example, temperatures, pressures and hourly gas space velocities in the range of from about (320-600°F) 160-350.5°C, (80-600 psi) 551-4137 kPa and 100-40,000 V/hr/V, expressed as standard volumes of the gaseous CO and H₂ mixture (0°C, 1 atm) per hour per volume of catalyst, respectively.

The invention will be further understood with reference to the Examples below.

### EXAMPLES

In preparing the hydrocarbon synthesis catalysts of the invention, air-dried zeolites, without a binder, were dehydrated in an inert gas flow for 4 hr. at 480-500°C. After cooling down to room temperature in a dry box, they were introduced as a powder under intensive stirring into a solution of cobalt nitrate or cobalt and magnesium nitrates, to which a precipitant solution of either sodium carbonate or ammonium carbonate had previously been added, with the solution then heated at its boiling point until CO₂ evolution ceased. The metal precipitate comprises one or more hydroxy carbonates, the structure of which is changed by boiling until the cessation of CO₂ evolution. This was followed by filtering the resulting precipitate and washing the resulting paste with hot water to remove nitrate ions, followed by extruding and drying in air at 105-120°C for from 65-170 minutes to form a catalyst precursor, which was then reduced in hydrogen at 380-400°C to form the catalyst. Preferably, the duration and the temperature at which the drying is conducted is chosen to achieve a satisfactory residual moisture content in the precursor prior to the reduction, as determined by the color of the precursor. For example, it has been found that Co-Mg-containing catalyst precursors with too low of a moisture content (e.g., less than about 6 wt. %) had a grey color as opposed to a violet color exhibited by precursors with a higher moisture content. This color change is indicative of a change in precursor structure, when too much moisture is removed from the precursor prior to reduction in hydrogen and is reflected in a catalyst with less CO conversion and C₅₊ hydrocarbon yield.

When used for hydrocarbon synthesis, at a synthesis gas pressure slightly above atmospheric, there was no decrease in activity over the 60-80 hr. runs in the laboratory fixed bed reactor. In preparing the catalysts, various single- and multivalent cation forms (Na, Ca, NH₄, H₂, etc.) of the zeolites are used as the supports or the zeolite component of the catalysts. Zeolites having a wide range of pore sizes were found to be effective and included, for example, X zeolites having a pore size of 8-9 , Y zeolites having a pore size of 7 , as well as the decationated form of Y zeolites and the hydrogen form of synthetic mordenite. The various zeolites were prepared from original sodium-containing zeolites, by ion exchange with 2-10 wt. % aqueous solutions of either chlorides or nitrates of the corresponding metals, in the case of cation forms, or solutions of either ammonium chloride or ammonium nitrate in the case of the hydrogen forms of the Y and mordenite zeolites. The latter may also be produced by direct treatment ofNa-mordenite with a 2N solution of HCl at room or elevated (80-90°C) temperature. Further, A zeolites having pores of 4-5 a re also effective.

### Example 1

A precipitant solution of 16 g of sodium carbonate dissolved in 80 g of water was added to a solution of 25 g of cobalt nitrate dissolved in 100 g of water, and the combined solutions heated to boiling. Boiling was continued until CO₂ evolution ceased. The resulting suspension was cooled and 10 g of powdered, dehydrated NaX zeolite (SiO₂/Al₂O₃ = 2.5) added to it to form a slurry. The zeolite had been dehydrated by being heated for 5 hours at 500°C, under dry, flowing N₂ and kept dry until it was added to the suspension. The slurry was stirred for 10 min. at room temperature and filtered. The filter cake was washed with hot water to remove nitrate ions, formed into a paste and extruded into 2x3-4 mm cylinders, which were dried in air at 110-120°C. The extrudates had a composition of 100Co:200NaX.

The dry extrudates were then loaded into a fixed bed laboratory reactor and reduced under flowing H₂ at 350-400°C (GHSV of 100 hr⁻¹) for 5 hr. A synthesis gas comprising a mixture of H₂ and CO having an H₂:CO mole ratio of 2:1, at a space velocity of 90 hr⁻¹, was passed through the catalyst bed at 190°C. The pressure was slightly above atmospheric. The gas contraction was 67.6 vol. % and this was also the amount of CO conversion. The synthesized hydrocarbons were successively passed through a cold water trap (17-22°C), followed by a dry ice trap. The C₅₊ liquid yield was 123 g/m³ (STP), with a gasoline fraction to heavier oil product liquid weight ratio of 1.4:1. By gasoline fraction is meant the liquid condensate in the dry ice trap, while the heavier oil was the liquid condensate in the cold water trap, which preceded the dry ice trap.

Thus, this catalyst exhibited excellent selectivity for gasoline production at a relatively low reaction temperature, with good CO conversion.

### Example 2

A hydrogen substituted Y zeolite was prepared by ion exchange according to well known procedures as described, for example, in US patents 3,287,282 and 3,503,901. A 0.01 N aqueous solution of NH₄Cl was prepared. Three mole equivalents of this solution was added to the sodium form of the Y zeolite, so that there was three mole equivalents of NH₄⁺ ion present, for each mole equivalent of Na⁺ ion in the zeolite, during the ion exchange reaction. The suspension was stirred and heated to 80°C. It was held at 80°C for 2 hours. Then the solution was decanted and the zeolite was dried in air at 100-120°C for 2 hours. The zeolite was then placed in a quartz reactor and calcined in flowing N₂ or He at 400°C for 4 hours. Then the zeolite was cooled and removed from the reactor. The extent of ion exchange (H⁺ for Na⁺) was 70%. The resulting HNaY zeolite (SiO₂/Al₂O₃ = 4.4:1, 70 % Na+ exchange) was stored under nitrogen in a dry box until use.

In this experiment, a precipitant solution of 16 g of sodium carbonate dissolved in 80 g of water, was added to a solution of 25 g of cobalt nitrate and 3.2 g of magnesium nitrate dissolved in 100 g of water, and the combined solutions heated to boiling. Boiling continued until CO₂ evolution ceased. The resulting suspension was cooled and 10 g of the powdered, dehydrated HNaY zeolite added to it to form a slurry. The slurry was stirred for 10 min. at room temperature and filtered. The filter cake was washed with hot water to remove nitrate ions, formed into a paste and extruded into 2x3-4 mm cylinders, which were dried in air at 110-120°C. The extrudates had a composition of 100Co:10MgO:200HNaY.

The dry extrudates were then loaded into a fixed bed laboratory reactor and reduced under flowing H₂ at 350-400°C (GHSV of 100 hr⁻¹) for 5 hr. A synthesis gas comprising a mixture of H₂ and CO having an H₂:CO mole ratio of 2:1, at a space velocity of 103 hr⁻¹, was passed through the catalyst bed at 180°C and a pressure slightly above atmospheric. The gas contraction was 83.5 vol. % and this was also the amount of CO conversion. The C₅₊ liquid yield was 145.5 g/m³ (STP).

Thus, this catalyst exhibited excellent selectivity for C₅₊ liquid production, at a relatively low reaction temperature, with even greater CO conversion than in Example 1.

### Example 3

The sodium form of a Y zeolite was mixed with a 10-fold mole excess of a 10 wt % aqueous CaCl₂ solution, for 4 hours at 80°C. Then the solution was decanted and the zeolite was dried in air at 100-120°C for 2 hours. The zeolite was then placed in a quartz reactor and calcined in flowing N₂ or He at 400°C for 4 hours. Then the zeolite was cooled and removed from the reactor. The extent of ion exchange (Ca⁺² for Na⁺) was 67% and the composition of the resulting zeolite was SiO₂/Al₂O₃ = 4.4:1.

A catalyst having a composition of 100Co:10MgO:200CaNaY (SiO₂/Al₂O₃ = 4.4, 67 % total Ca+, Na+ exchange) was prepared, extruded and reduced using the same procedure used in Example 2, and then loaded into the fixed bed laboratory reactor. The CaNaY zeolite added to the combined solutions after CO₂ removal, was dehydrated as in Example 2. The same 2:1 H₂ to CO mole ratio synthesis gas composition was again used. At the same reaction conditions of a temperature of 180°C, a pressure slightly above atmospheric and a GHSV of 100 hr⁻¹, the gas contraction was 84 %, which meant a CO conversion of 84 %, and the C₅₊ yield of synthesized hydrocarbons was 142 g/cm³ (STP).

### Example 4

The sodium form ofmordenite zeolite was mixed with a 3-fold mole excess of an aqueous, 2N HCl solution at 95°C. Then the solution was decanted and the zeolite was dried in air at 100-120°C for 2 hours. The zeolite was then placed in a quartz reactor and calcined in flowing N₂ or He at 400°C for 4 hours. Then the zeolite was cooled and removed from the reactor. The extent of ion exchange (H⁺ for Na⁺) was 100% and the SiO₂ to Al₂O₃ mole ratio of the resulting H- mordenite zeolite was 10.5.

A catalyst using this H-mordenite (HM) type of zeolite was prepared using the procedure of Example 2 and had a composition of 100Co:10MgO:200HM (SiO₂/Al₂O₃ = 10.5, 100 % exchange). The HM had been dehydrated prior to being added to the combined solutions after CO₂ removal. Except for a temperature of 191°C, the other reaction conditions were the same as in Example 2. The gas contraction was 86.6 vol. % and the liquid hydrocarbon yield was 110 g/m³ (STP).

## Claims

1. A process for forming a catalyst comprising at least one catalytic metal component selected from Group VIII of the Periodic Table, optionally one or more promoter components and at least one zeolite component, comprises:
(i) adding a carbonate ion precipitant, under mixing conditions, to an aqueous solution of one or more catalytic metal salts, to form a slurry comprising a precipitate comprising a first hydroxy metal carbonate of said one or more catalytic metal components;
(ii) heating said slurry to remove CO₂ to change said first hydroxy metal carbonate to a second hydroxy metal carbonate and form a CO₂-reduced, second slurry containing said second hydroxy metal carbonate precipitate;
(iii) compositing said second hydroxy metal carbonate precipitate from said CO₂-reduced slurry with said at least one zeolite component to form a composite of said precipitate and zeolite;
(iv) drying said composite to form a catalyst precursor containing between 6 to 15 wt. % residual moisture, and
(v) reducing said precursor with hydrogen to form said catalyst.

2. A process according to claim 1 wherein an aqueous solution of one or more carbonate or bicarbonate salts is added to said catalytic metal salt solution, to provide said carbonate ion precipitant.

3. A process according to claim 2 wherein said slurry is heated until said CO₂ evolution ceases.

4. A process according to claim 1 wherein said zeolite component is dehydrated prior to being contacted with said CO₂-reduced slurry.

5. A process according to claim 1 wherein said zeolite comprises at least one of a Y zeolite, an HY zeolite and an HZSM-5 zeolite.

6. A process according to claim 1 wherein said catalyst contains one or more promoter metal components.

7. A process according to claim 1 wherein said catalytic metal comprises cobalt.

8. A process according to claim 6 wherein said promoter metal comprises at least one of Re, Ru, Fe, Ni, Th, Zr, Hf, U, Mg and La.

9. A process according to claim 1 wherein said zeolite comprises a stabilized zeolite.

10. A process according to claim 8 wherein said promoter metal is selected from the group consisting essentially of Re, Ru, Fe, Ni, Th, Zr, Hf, U, Mg and La, and mixture thereof.

## Patentansprüche

1. Verfahren zur Herstellung von Katalysator, der mindestens eine katalytische Metallkomponente ausgewählt aus der Gruppe VIII des Periodensystems, gegebenenfalls eine oder mehrere Promoterkomponenten und mindestens eine Zeolithkomponente umfasst, bei dem
(i) ein Carbonation-Ausfällungsmittel unter Mischbedingungen zu einer wässrigen Lösung von einem oder mehreren katalytischen Metallsalzen gegeben wird, um eine Aufschlämmung zu bilden, die einen Niederschlag umfasst, der ein erstes Hydroxymetallcarbonat der einen oder mehreren katalytischen Metallkomponenten umfasst,
(ii) die Aufschlämmung erwärmt wird, um CO₂ zu entfernen, um das erste Hydroxymetallcarbonat in ein zweites Hydroxymetallcarbonat umzuwandeln und eine CO₂-reduzierte zweite Aufschlämmung zu bilden, die den zweiten Hydroxymetallcarbonat-Niederschlag enthält,
(iii) der zweite Hydroxymetallcarbonat-Niederschlag aus der CO₂-reduzierten Aufschlämmung mit der mindestens einen Zeolithkomponente gemischt wird, um ein Komposit aus dem Niederschlag und dem Zeolithen zu bilden,
(iv) das Komposit getrocknet wird, um einen Katalysatorvorläufer zu bilden, der zwischen 6 und 15 Gew.-% Restfeuchtigkeit enthält, und
(v) der Vorläufer mit Wasserstoff reduziert wird, um den Katalysator zu bilden.

2. Verfahren nach Anspruch 1, bei dem eine wässrige Lösung von einem oder mehreren Carbonat- oder Bicarbonatsalzen zu der katalytischen Metallsalzlösung gegeben wird, um das Carbonation-Ausfällungsmittel zu liefern.

3. Verfahren nach Anspruch 2, bei dem die Aufschlämmung erwärmt wird, bis die CO₂-Entwicklung endet.

4. Verfahren nach Anspruch 1, bei dem die Zeolithkomponente dehydratisiert wird, bevor sie mit der CO₂-reduzierten Aufschlämmung kontaktiert wird.

5. Verfahren nach Anspruch 1, bei dem der Zeolith mindestens einen von Y-Zeolith, HY-Zeolith und HZSM-5-Zeolith umfasst.

6. Verfahren nach Anspruch 1, bei dem der Katalysator eine oder mehrere Promotermetallkomponenten enthält.

7. Verfahren nach Anspruch 1, bei dem das katalytische Metall Kobalt umfasst.

8. Verfahren nach Anspruch 6, bei dem das Promotermetall mindestens einen von Re, Ru, Fe, Ni, Th, Zr, Hf, U, Mg und La umfasst.

9. Verfahren nach Anspruch 1, bei dem der Zeolith stabilisierten Zeolith umfasst.

10. Verfahren nach Anspruch 8, bei dem das Promotermetall ausgewählt ist aus der Gruppe bestehend im Wesentlichen aus Re, Ru, Fe, Ni, Th, Zr, Hf, U, Mg und La und Mischungen davon.

## Revendications

1. Procédé pour former un catalyseur comprenant au moins un composant de métal catalytique choisi dans le groupe VIII du tableau périodique, éventuellement un ou plusieurs composants promoteurs et au moins un composant de zéolite, comprenant les étapes suivantes :
(i) on ajoute un précipitant d'ion carbonate, dans des conditions de mélange, à une solution aqueuse d'un ou plusieurs sels de métaux catalytiques, pour former une suspension comprenant un précipité comprenant un premier hydroxycarbonate de métal dudit un ou plusieurs composants de métaux catalytiques ;
(ii) on chauffe ladite suspension pour éliminer du CO₂ afin de changer ledit premier hydroxycarbonate de métal en un second hydroxycarbonate de métal et former une seconde suspension à teneur réduite en CO₂, contenant ledit second précipité d'hydroxycarbonate de métal ;
(iii) on combine ledit second précipité d'hydroxycarbonate de métal de ladite suspension à teneur réduite en CO₂ avec ledit au moins un composant de zéolite pour former un composite dudit précipité et de ladite zéolite ;
(iv) on sèche ledit composite pour former un précurseur de catalyseur contenant 6 à 15 % en poids d'humidité résiduelle ; et
(v) on réduit ledit précurseur avec de l'hydrogène pour former ledit catalyseur.

2. Procédé selon la revendication 1, dans lequel une solution aqueuse d'un ou plusieurs sels de carbonates ou bicarbonates est ajoutée à ladite solution de sel de métal catalytique pour fournir ledit precipitant d'ion carbonate.

3. Procédé selon la revendication 2, dans lequel ladite suspension est chauffée jusqu'à ce que ledit dégagement de CO₂ cesse.

4. Procédé selon la revendication 1, dans lequel ledit composant de zéolite est deshydraté avant d'être mis en contact avec ladite suspension à teneur réduite en CO₂.

5. Procédé selon la revendication 1, dans lequel ladite zéolite comprend au moins une zéolite parmi les zéolites Y, HY et HZSM-5.

6. Procédé selon la revendication 1, dans lequel ledit catalyseur contient un ou plusieurs composants métalliques promoteurs.

7. Procédé selon la revendication 1, dans lequel ledit métal catalytique comprend du cobalt.

8. Procédé selon la revendication 6, dans lequel ledit métal promoteur comprend au moins un des suivants : Re, Ru, Fe, Ni, Th, Zr, Hf, U, Mg et La.

9. Procédé selon la revendication 1, dans lequel ladite zéolite comprend une zéolite stabilisée.

10. Procédé selon la revendication 8, dans lequel ledit métal promoteur est choisi dans le groupe constitué essentiellement de Re, Ru, Fe, Ni, Th, Zr, Hf, U, Mg et La et de leurs mélanges.
